# EUROPEAN PATENT SPECIFICATION

(11) **EP 1 066 260 B1**
(45) Date of publication and mention of the grant of the patent: **16.06.2004**
(21) Application number: 98964189.9
(22) Date of filing: 21.12.1998
(51) Int. Cl.: C07D 213/06, C07D 213/08, C07D 213/14

(54) **PYRIDINE/PICOLINE PRODUCTION PROCESS**
HERSTELLUNG VON PYRIDINEN/PICOLINEN
PROCEDE DE PRODUCTION DE PYRIDINE/PICOLINE

(30) Priority: 31.12.1997 US 2363
(43) Date of publication of application: 10.01.2001
(73) Proprietor: EXXONMOBIL OIL CORPORATION, Fairfax, VA 22037 (US)
(72) Inventor: CHESTER, Arthur, Warren, Cherry Hill, NJ 08003 (US); HAN, Scott, Lawrenceville, NJ 08648 (US); MAZZONE, Dominick, Nicholas, Wenonah, NJ 08090 (US); VENKAT, Chaya, Rao, Princeton, NJ 08540 (US)
(74) Representative: Dew, Melvyn John
(86) International application number: PCT/US1998/027197
(87) International publication number: WO 1999/033808

(56) References cited:
- US-A- 4 220 783
- US-A- 5 780 635
- GRAYSON J.I. et al., "An Improved Liquid-Phase Synthesis of Simple Alkylpyridines ", SWITZERLAND: HELVETICA CHIMICA ACTA, 1984, Vol. 7, pages 2100-2110, XP002916281

## Description

This invention relates to a process for producing pyridine and/or picoline.

Pyridine is an important intermediate in the manufacture of agricultural chemicals, e.g., herbicides and pesticides, and pharmaceuticals, and is also useful as a solvent in the polymer and textile industries. Important derivatives of pyridine include, for example, nicotinic acid and nicotinamide (vitamins essential for human health), chlorpheniramine (an antihistamine), cetylpyridinium (a germicide and antiseptic), isoniazid (an important antitubercular drug), and Paraquat® (a herbicide).

Pyridines having one methyl group attached to the ring structure are called methylpyridines or picolines, and include 2- or α-picoline, 3- or β-picoline, and 4- or γ-picoline.

Pyridine and picolines can be obtained as by-products of the coal tar industry or coke manufacture. However, pyridine is found in only small amounts in coal tar, and a preferred method of obtaining pyridine is by chemical synthesis. Chemical synthesis typically relies on a catalytic gaseous reaction (condensation) between ammonia (or amines) and carbonyl compounds such as aldehydes or ketones. However, these chemical synthesis methods have historically suffered from disadvantages of low yields and poor selectivity, and short operation cycle and catalyst lifetime.

The term "base synthesis" is known and used in the field of pyridine chemistry to identify synthetic processes by which bases of pyridine and its alkylated derivatives are prepared by reacting aldehydes and/or ketones with ammonia in the gas phase using a heterogeneous catalyst. For example, the reaction of acetaldehyde with ammonia in the presence of heterogenous catalysts at 350 to 550°C yields 2- and 4-methylpyridines (α- and γ-picolines). As another example, acetaldehyde and formaldehyde, can be reacted with ammonia to yield pyridine and 3-methylpyridine. Such pyridine synthesis methods are described, for example, in U.S. Patent No. 4,675,410 to Feitler and U.S. Patent No. 4,220,783 to Chang et al.

Reaction of acetaldehyde or certain other low molecular weight aldehydes and ammonia either in the absence or presence of methanol and/or formaldehyde to yield pyridine and alkyl derivatives thereof has been carried out in the presence of amorphous silica-alumina composites containing various promoters. See, for example, U.S. Patent Nos. 2,807,618 and 3,946,020. The yields of desired products using the latter catalysts have been poor. Alkylpyridines have also been synthesized, as reported in *Advances in Catalysis,* 18:344 (1968), by passing gaseous acetaldehyde and ammonia over the crystalline aluminosilicates NaX and H-mordenite. While initial conversion utilizing these materials as catalysts was high, catalyst deactivation by coking was rapid, providing a commercially unattractive system, characterized by poor catalytic stability.

Synthetic crystalline zeolites having an intermediate pore size as measured by the Constraint Index of the zeolite being between 1 and 12, e.g., ZSM-5, have been found to provide commercially useful yields and product selectivities. U.S. Patent No. 4,220,783 was pioneer in this discovery, teaching synthesis of pyridine and alkylpyridines by reacting ammonia and a carbonyl reactant which is an aldehyde containing 2 to 4 carbon atoms, a ketone containing 3 to 5 carbon atoms or mixtures of the aldehydes and/or ketones under effective conditions in the presence of a catalyst comprising a crystalline aluminosilicate zeolite having been ion exchanged with cadmium and having a silica to alumina ratio of at least 12, and a Constraint Index within the range of 1 to 12.

Use of a ZSM-5 catalyst component in a fluidized or otherwise movable bed reactor is taught in U.S. Patent No. 4,675,410. U.S. Patent No. 4,886,179 teaches synthesis of pyridine by reaction of ammonia and a carbonyl compound, preferably with added hydrogen, over catalyst comprising a crystalline aluminosilicate zeolite which has been ion exchanged with a Group VIII metal of the Periodic Table. The crystalline aluminosilicate zeolite has a silica to alumina mole ratio of at least 15, preferably 30 to 200, a Constraint Index of from 4 to 12, e.g., ZSM-5, and the process provides a high and selective yield of pyridine.

U.S. Patent No. 5,013,843 teaches addition of a third aldehyde or ketone to a binary mixture of aldehydes and/or ketones used in preparing mixtures of pyridine and alkyl-substituted pyridines in large scale continuous processes. In a preferred system, propionaldehyde is added to a binary mixture of acetaldehyde and formaldehyde to produce beta-pyridine and pyridine. The catalyst for this process is a crystalline aluminosilicate zeolite in the acidic form having a Constraint Index of from 1 to 12, e.g., ZSM-5.

However, despite recent advances, existing processes for producing pyridine and picolines suffer from the disadvantage that their selectivity to the desired pyridine and alkylpyridine is poor, making the processes unacceptable from a commercial perspective. Accordingly, an object of the present invention is to provide an improved pyridine and picoline synthetic process in which high yields and high purities of desirable products can be achieved in a cost- and time-efficient manner.

Accordingly the invention resides in a process for producing pyridine or picoline compounds, comprising:
**(a)** reacting a reactant feedstream comprising ammonia and at least one C₁₋₅ carbonyl reactant in the presence of a molecular sieve catalyst to produce a primary product stream comprising pyridine or picoline products and polyalkylpyridines;
**(b)** isolating from the primary product stream a product fraction comprising pyridine or picoline products, and a polyalkylpyridine fraction comprising polyalkylpyridines; and
**(c)** contacting the polyalkylpyridine fraction with a molecular sieve catalyst to produce a secondary product stream comprising pyridine or picoline products.

The process of the invention substantially reduces the net make of polyalkylpyridines or higher molecular weight aromatic hydrocarbon species and increases the yield of the desired pyridine and picoline products. Preferably, the net amount of polyalkylpyridines and higher molecular weight aromatic species in the product fraction and the secondary product stream together comprise less than 5wt.%, and more preferably less than 2 wt.%, of the total product yield.

Optionally, the converting step (c) comprises recirculating the polyalkylpyridine fraction to the catalyst used for the reacting step (a), so that the contacting step (c) is effected under the same reaction conditions as the reacting step (a).

The present invention provides a "base synthesis" route to the production of pyridine or alkylpyridine derivatives in which a carbonyl compound is initially reacted with ammonia in the gas phase using a heterogeneous, molecular sieve catalyst. The product of the base synthesis reaction is separated into a pyridine and/or picoline fraction, which is recovered, and a polyalkylpyridine fraction which is contacted with a molecular sieve catalyst to produce additional pyridine and/or picoline products.

The carbonyl reactant used in the base synthesis reaction is a hydrocarbon compound containing 1 to 5 carbon atoms and at least one carbonyl moiety. The carbonyl reactant taking part in the catalytic reaction described herein may be formaldehyde, an aldehyde containing 2 to 4 carbon atoms, a ketone containing 3 to 5 carbon atoms or mixtures thereof. Representative reactant aldehydes include acetaldehyde, propionaldehyde, acrolein, butyraldehyde, and crotonaldehyde. Representative reactant ketones include acetone, methyl ethyl ketone, diethyl ketone, and methyl propyl ketone. The carbonyl reactant can be present as a solution in water, e.g., formalin which is a solution of formaldehyde in water, with a small amount of methanol to aid solubility.

The carbonyl reactant may comprise a mixture of two or more carbonyl compounds. If a mixture is used, each carbonyl component in the mixture is preferably present in a predetermined amount relative to the other carbonyl components. For example, when the at least one carbonyl reactant is a mixture of formaldehyde and acetaldehyde, the two components are preferably present in a formaldehyde/acetaldehyde mole ratio of from 0.2 to 1.0, more preferably from 0.4 to 0.8. Alternatively, mixtures of acetaldehyde and acrolein will typically have an acetaldehyde/acrolein mole ratio of from 0.7 to 1.25. Other mixtures of carbonyl reactants can be similarly formulated to selectively control the product of the base synthesis. Preferably the carbonyl reactant comprises a mixture of formaldehyde and acetaldehyde and the primary product of the base synthesis reaction is pyridine and β-picoline.

The mole ratio of ammonia to carbonyl reactant (NH₃/CO) in the reaction mixture employed will generally be between 0.5 and 30, preferably between 0.5 and 10, and more preferably between 1 and 5.

Hydrogen gas (H₂) may, if desired, be added to the reaction, e.g., at the rate of from 0 (no added hydrogen) to an H₂/carbonyl reactant (H₂/CO) mole ratio of 5.0, preferably from 0.1 to 1.0.

The reaction conditions for performing the base synthesis reaction between the ammonia and the at least one carbonyl compound include:
**(a**) a temperature of 285 to 600°C, preferably 340 to 550°C;
**(b)** a pressure of 20 to 2,000 kPa (0.2 to 20 atm), preferably 80 to 1,000 kPa (0.8 to 10 atm); and
**(c)** a gas hourly space velocity (GHSV) of 200 to 20,000 hr⁻¹, preferably 300 to 5,000 hr⁻¹.

At the completion of the base synthesis reaction, the recovered product is separated into its desired components by any feasible means, e.g., by fractionation, to recover a product containing pyridine and/or one or more picoline compounds. Of the picoline compounds selectively produced by way of the present invention, 3-picoline is an important intermediate in the manufacture of 3-pyridinecarboxylic acid, i.e., nicotinic acid, and other medicinal, agricultural, and chemical products. The process of the invention can be further modified to accommodate the synthesis of 3-pyridinecarboxylic acid, by recovering the 3-picoline product and contacting the recovered 3-picoline with an oxidative reagent such as KMnO₄.

The process of the invention has the advantage that the make of polyalkylpyridines and/or other higher molecular weight aromatic species is substantially reduced as compared to conventional processes, while the yield of the desired pyridine or picoline products is increased. This is achieved by contacting the polyalkylpyridine by-products in a secondary reaction with a molecular sieve catalyst. By "polyalkylpyridine by-products" is meant pyridine derivatives having two or more alkyl groups attached to the ring structure. Such compounds are exemplified by lutidine and collidine. In a conventional single-pass reaction, these polyalkylpyridine by-products, as well as other higher molecular weight aromatic species (such as alkylated polynuclear aromatic structures) are produced in amounts of up to 20 wt.% or more, which makes such processes highly inefficient. The process of the invention, by contrast, has been found to reduce the total make of polyalkylpyridines and higher molecular weight aromatic species to less than 5 wt.%, often less than 2 wt.%, and usually less than 1 wt.%.

The secondary reaction converts the polyalkylpyridines or other higher molecular weight aromatic species in the polyalkylpyridine fraction to secondary product enriched for pyridine or picoline products. The catalyst and the conversion conditions used for the secondary reaction may be the same as or different from those used for the primary reaction, although the conversion conditions for the secondary reaction will generally be within the same approximate ranges as those specified abobe for the base synthesis reaction. Preferably, the secondary reaction is effected in the same reactor, using the same catalyst as, the base synthesis reaction by recycling part or all of the polyalkylpyridine fraction back to the primary reactor.

### Catalyst System

The base synthesis reaction and the polyalkylpyridine conversion reaction are performed over a molecular sieve catalyst. Preferred molecular sieve catalysts are those having an intermediate pore size characterized by a Constraint Index 1 to 12. Constraint Index and the method by which it is determined are described in U.S. Patent No. 4,016,218. Examples of suitable intermediate pore size molecular sieves include ZSM-5 (U.S. Patent No. 3,702,886 and Re. 29,948); ZSM-11 (U.S. Patent No. 3,709,979); ZSM-12 (U.S. Patent No. 3,832,449); ZSM-22 (U.S. Patent No. 4,556,447); ZSM-23 (U.S. Patent No. 4,076,842); ZSM-35 (U.S. Patent No. 4,016,245); ZSM-48 (U.S. Patent No. 4,397,827); ZSM-57 (U.S. Patent No. 4,046,685); and ZSM-58 (U.S. Patent No. 4,417,780).

Other useful catalyst materials include MCM-22 (U.S. Patent No. 4,954,325), MCM-36 (U.S. Patent No. 5,250,277), MCM-49 (U.S. Patent No. 5,236,575) and MCM-56 (U.S. Patent No. 5,362,697). SAPO-5, SAPO-11, zeolite X, zeolite Y, and zeolite Beta are also examples of molecular sieve materials that can also be used according to the invention.

Naturally occurring clays that can be composited with the molecular sieve include those of the montmorillonite and kaolin families, which families include the subbentonites and the kaolins commonly known as Dixie, McNamee, Georgia, and Florida clays, or others in which the main mineral constituent is halloysite, kaolinite, dickite, nacrite, or anauxite. Suitable clay materials include, by way of example, bentonite and kieselguhr. Such clays can be used in the raw state as originally mined or initially subjected to calcination, acid treatment, or chemical modification.

The relative proportion of suitable crystalline molecular sieve to the total composition of catalyst and binder or support may be from 1 wt.% to 99 wt.%, preferably from 30 wt.% to 90 wt.%, and more preferably from 50 wt.% to 80 wt.%, of the composition.

A hydrogenation-dehydrogenation functional metal can be incorporated into the catalyst of the invention. The amount of the functional metal is suitably from 0.001 wt.% to 10 wt.%, preferably from 0.05 wt.% to 5 wt.%, more preferably from 0.1 wt.% to 2 wt.%, based on the total weight of the modified catalyst. Examples of suitable hydrogenation-dehydrogenation metals include Group 8, 9, and 10 metals (i.e., Pt, Pd, Ir, Rh, Os, Ru, Ni, Co, and Fe), Group 7 metals (i.e., Mn, Tc, and Re), Group 6 metals (i.e., Cr, Mo, and W), Group 15 metals (i.e., Sb and Bi), Group 14 metals (i.e., Sn and Pb), Group 13 metals (i.e., Ga and In), Group 11 metals (i.e., Cu, Ag, and Au), and Group 12 metals (i.e., Zn, Cd, and Hg). Noble metals (i.e., Pt, Pd, Ir, Rh, Os, Re, Ru, Mo, and W) are preferred.

### COMPAPATIVE EXAMPLE 1

A base synthesis reaction to produce pyridine and picoline products was performed using as the catalyst a 40% HZSM-5 zeolite spray-dried in a silica-alumina-clay matrix. In a typical run, 5-10 mL of 20/40 mesh catalyst was charged to a quartz reactor and heated to 440 to 454°C (825 to 850°F) under an N₂ purge. The feed consisted of acetaldehyde, formaldehyde, ammonia, and hydrogen in the following molar ratios: acetaldehyde = 1.4; formaldehyde = 1.0; ammonia = 3.6; hydrogen = 1.6. The feed was passed over the catalyst at 580 hr⁻¹ GHSV (NH₃), and the effluent was analyzed by gas chromatography (GC) using quinoline as an internal standard. Water content in the product was determined by Karl-Fischer titration. The yields of the various products of this reaction are given in Table 1.

### EXAMPLE 1

The pyridine and picoline products in the effluent of the reaction described in comparative Example 1 were distilled off and collected, and the aqueous layer was removed. The heavier N-containing components were recycled back into the reactor. Additional catalyst was added as needed to increase the conversion of polymethylpyridines. The yields of the various products following this reaction are given in Table 1.

**TABLE 1**

| | **Comparative Example 1** **(No Recycle)** | **Example 1** **(With Recycle)** |
|---|---|---|
| Temperature (°C) | 440 | 440 |
| Catalyst Charge (mL) | 5.0 | 7.0 |
| Run Time (min) | 120 | 120 |
| | | |
| Acetaldehyde Conversion (%) | 85.9 | 86 |
| Formaldehyde Conversion (%) | 79.1 | 80 |
| Yield of N-Products¹ (%) | 43.1 | 43 |
| | | |

| Selectivities (%) | | |
|---|---|---|
| Pyridine | 38.7 | 50 |
| Picoline | 22.1 | 30 |
| Polyalkylpyridines | 18.1 | <1 |
| Higher MW Products, tars, etc. | 21.1 | 20 |

| | | |
|---|---|---|
| ¹ Wt N-containing products/wt acetaldehyde feed | | |

Table 1 clearly illustrates that the process of the invention (Example 1) can substantially increase the process selectivity for pyridine and picolines as compared to the prior art single pass process comparative (Example 1). Polyalkylpyridine by-product yield is substantially reduced, from 18.1% to less than 1%. Note that, for comparison purposes, the acetaldehyde conversions for each example are accomplished by increasing the catalyst charge to the reactor. Accordingly, the process of the invention achieves a substantial increase in efficiency on the basis of the total yield of pyridine and picoline products from a given amount of feed.

## Claims

1. A process for producing pyridine or picoline compounds, comprising the steps of:
**(a)** reacting a reactant feedstream comprising ammonia and at least one C₁₋₅ carbonyl reactant in the presence of a molecular sieve catalyst to produce a primary product stream comprising pyridine or picoline products and polyalkylpyridines;
**(b)** isolating from the primary product stream a product fraction comprising pyridine or picoline products, and a polyalkylpyridine fraction comprising polyalkylpyridines; and
**(c)** contacting the polyalkylpyridine fraction with a molecular sieve catalyst to produce a secondary product stream comprising pyridine or picoline products.

2. The process according to Claim 1, wherein the conditions of steps (a) and (b) each comprise a temperature of 285 to 600°C; a pressure of 20 to 2,000 kPa (0.2 to 20 atm); and a GHSV of 200 to 20,000 hr⁻¹.

3. The process according to Claim 1, wherein the conditions of steps (a) and (b) each comprise a temperature of 340 to 550°C; a pressure of 80 to 1,000 kPa (0.8 atm to 10 atm); and a GHSV of 300 to 5,000 hr⁻¹.

4. The process according to Claim 1, wherein the molecular sieve is selected from ZSM-5, ZSM-11, ZSM-12, ZSM-22, ZSM-23, ZSM-35, ZSM-48, ZSM-57, ZSM-58, MCM-22, MCM-36, MCM-49, MCM-56, SAPO-5, SAPO-11, zeolite Beta, zeolite X, and zeolite Y.

5. The process according to Claim 1, wherein the mole ratio of the ammonia to the at least one carbonyl reactant in the reactant feedstream is from 0.5 to 10.

6. The process according to Claim 1, wherein the at least one carbonyl reactant comprises acetaldehyde and formaldehyde.

7. The process according to Claim 1, further comprising co-feeding hydrogen with the reactant feedstream.

8. The process according to Claim 1, wherein the converting step (c) comprises recirculating the polyalkylpyridine fraction to the catalyst used for the reacting step (a), and wherein the conversion is accomplished under the synthesis reaction conditions of the reacting step (a).

## Patentansprüche

1. Verfahren zur Herstellung von Pyridin- oder Picolinverbindungen mit den Schritten:
(a) Umsetzen eines Ammoniak und mindestens einen C₁₋₅-Carbonylreaktanten umfassenden Reaktanteinsatzmaterialstroms in Gegenwart eines Molekularsiebkatalysators, um einen Pyridin- oder Picolinprodukte und Polyalkylpyridine umfassenden Primärproduktstrom herzustellen,
(b) Isolieren einer Pyridin- oder Picolinprodukte umfassenden Produktfraktion und einer Polyalkylpyridine umfassenden Polyalkylpyridinfraktion aus dem Primärproduktstrom und
(c) Inkontaktbringen der Polyalkylpyridinfraktion mit einem Molekularsiebkatalysator, um einen Pyridin- oder Picolinprodukte umfassenden Sekundärproduktstrom herzustellen.

2. Verfahren nach Anspruch 1, bei dem die Bedingungen der Schritte (a) und (b) jeweils eine Temperatur von 285 bis 600 °C, einen Druck von 20 bis 2000 kPa (0,2 bis 20 atm) und einen GHSV von 200 bis 20 000 h⁻¹ umfassen.

3. Verfahren nach Anspruch 1, bei dem die Bedingungen der Schritte (a) und (b) jeweils eine Temperatur von 340 bis 550 °C, einen Druck von 80 bis 1000 kPa (0,8 atm bis 10 atm) und einen GHSV von 300 bis 5000 h⁻¹ umfassen.

4. Verfahren nach Anspruch 1, bei dem das Molekularsieb ausgewählt ist aus ZSM-5, ZSM-11, ZSM-12, ZSM-22, ZSM-23, ZSM-35, ZSM-48, ZSM-57, ZSM-58, MCM-22, MCM-36, MCM-49, MCM-56, SAPO-5, SAPO-11, Zeolith β, Zeolith X und Zeolith Y.

5. Verfahren nach Anspruch 1, bei dem das Molverhältnis von Ammoniak zu dem mindestens einen Carbonylreaktanten in dem Reaktanteinsatzmaterialstrom 0,5 bis 10 beträgt.

6. Verfahren nach Anspruch 1, bei dem der mindestens eine Carbonylreaktant Acetaldehyd und Formaldehyd umfasst.

7. Verfahren nach Anspruch 1, bei dem ferner Wasserstoff mit dem Reaktanteinsatzmaterialstrom co-eingespeist wird.

8. Verfahren nach Anspruch 1, bei dem der Umwandlungsschritt (c) das Rezirkulieren der Polyalkylpyridinfraktion zu dem für den Umsetzungsschritt (a) verwendeten Katalysator umfasst und bei dem die Umwandlung unter den Synthesereaktionsbedingungen des Umsetzungsschritts (a) durchgeführt wird.

## Revendications

1. Procédé pour la production de dérivés de pyridine ou de picoline, comprenant les étapes consistant :
(a) à faire réagir un courant réactionnel d'alimentation comprenant de l'ammoniac et au moins un corps réactionnel à fonction carbonyle en C₁ à C₅ en présence d'un catalyseur à base de tamis moléculaire pour produire un courant de produits primaires comprenant des produits consistant en dérivés de pyridine ou de picoline et des polyalkylpyridines ;
(b) à isoler du courant de produits primaires une fraction de produits comprenant des produits consistant en dérivés de pyridine ou de picoline et une fraction de polyalkylpyridines, comprenant des polyalkylpyridines ; et
(c) à mettre en contact la fraction de polyalkylpyridines avec un catalyseur à base de tamis moléculaire pour produire un courant de produits secondaires comprenant des produits consistant en dérivés de pyridine ou de picoline.

2. Procédé suivant la revendication 1, dans lequel les conditions des étapes (a) et (b) comprennent pour chacune une température comprise dans l'intervalle de 285 à 600°C ; une pression comprise dans l'intervalle de 20 à 2000 kPa (0,2 à 20 atm) et une VSHG comprise dans l'intervalle de 200 à 20 000 h⁻¹.

3. Procédé suivant la revendication 1, dans lequel les conditions des étapes (a) et (b) comprennent pour chacune une température comprise dans l'intervalle de 340 à 550°C ; une pression comprise dans l'intervalle de 80 à 1000 kPa (0,8 atm à 10 atm) et une VSHG comprise dans l'intervalle de 300 à 500 h⁻¹.

4. Procédé selon la revendication 1, dans lequel le tamis moléculaire est choisi entre les ZSM-5, ZSM-11, ZSM-12, ZSM-22, ZSM-23, ZSM-35, ZSM-48, ZSM-57, ZSM-58, MCM-22, MCM-36, MCM-49, MCM-56, SAPO-5, SAPO-11, la zéolite bêta, la zéolite X et la zéolite Y.

5. Procédé selon la revendication 1, dans lequel le rapport molaire de l'ammoniac audit au moins un corps réactionnel à fonction carbonyle dans le courant de corps réactionnels d'alimentation est compris dans l'intervalle de 0,5 à 10.

6. Procédé selon la revendication 1, dans lequel ledit au moins un corps réactionnel à fonction carbonyle comprend l'acétaldéhyde et le formaldéhyde.

7. Procédé suivant la revendication 1, comprenant en outre la co-introduction d'hydrogène avec le courant de corps réactionnels d'alimentation.

8. Procédé suivant la revendication 1, dans lequel l'étape de conversion (c) comprend la recirculation de la fraction de polyalkylpyridines au catalyseur utilisé pour l'étape de réaction (a), et dans lequel la conversion est effectuée dans les conditions réactionnelles de synthèse de l'étape de réaction (a).
